# EUROPEAN PATENT SPECIFICATION

(11) **EP 3 422 388 B1**
(45) Date of publication and mention of the grant of the patent: **05.04.2023**
(21) Application number: 18179957.8
(22) Date of filing: 26.06.2018
(51) Int. Cl.: H01J 49/00, G01N 33/92

(54) **LIPID-ANALYZING METHOD USING MASS SPECTROMETRY AND MASS SPECTROMETER**
VERFAHREN ZUR LIPIDANALYSE MITTELS MASSENSPEKTROMETRIE UND MASSENSPEKTROMETER
PROCÉDÉ D'ANALYSE DE LIPIDES UTILISANT LA SPECTROMÉTRIE DE MASSE ET SPECTROMÈTRE DE MASSE

(30) Priority: 28.06.2017 JP 2017125760
(43) Date of publication of application: 02.01.2019
(73) Proprietor: Shimadzu Corporation, Kyoto 604-8511 (JP)
(72) Inventor: TAKAHASHI, Hidenori, Kyoto-shi,, Kyoto 604-8511 (JP); SEKIYA, Sadanori, Kyoto-shi,, Kyoto 604-8511 (JP)
(74) Representative: Mewburn Ellis LLP

(56) References cited:
- US-A1- 2009 152 458
- US-A1- 2015 097 114
- US-A1- 2016 372 311
- LIANG ET AL: "Electron Transfer Dissociation of Doubly Sodiated Glycerophosphocholine Lipids", JOURNAL OF THE AMERICAN SOCIETY FOR MASS SPECTROMETRY, ELSEVIER SCIENCE INC, US, vol. 18, no. 10, 1 October 2007 (2007-10-01), pages 1783-1788, XP022262070, ISSN: 1044-0305, DOI: 10.1016/J.JASMS.2007.07.013
- LI PENGFEI ET AL: "Multistage mass spectrometry of phospholipids using collision-induced dissociation (CID) and metastable atom-activated dissociation (MAD)", INTERNATIONAL JOURNAL OF MASS SPECTROMETRY, ELSEVIER SCIENCE PUBLISHERS, AMSTERDAM, NL, vol. 403, 17 March 2016 (2016-03-17), pages 1-7, XP029622070, ISSN: 1387-3806, DOI: 10.1016/J.IJMS.2016.02.010
- KEIJI G ASANO ET AL: "Thermal dissociation in the quadrupole ion trap: ions derived from leucine enkephalin", INTERNATIONAL JOURNAL OF MASS SPECTROMETRY., vol. 185-187, 12 April 1999 (1999-04-12), pages 207-219, XP055525073, NL ISSN: 1387-3806, DOI: 10.1016/S1387-3806(98)14116-7
- HUONG T. PHAM ET AL: "Differentiation of Complex Lipid Isomers by Radical-Directed Dissociation Mass Spectrometry", ANALYTICAL CHEMISTRY, vol. 84, no. 17, 22 August 2012 (2012-08-22), pages 7525-7532, XP055431498, US ISSN: 0003-2700, DOI: 10.1021/ac301652a
- ROBERT E. DEIMLER ET AL: "Radical-induced fragmentation of phospholipid cations using metastable atom-activated dissociation mass spectrometry (MAD-MS)", INTERNATIONAL JOURNAL OF MASS SPECTROMETRY., vol. 390, 18 August 2015 (2015-08-18), pages 178-186, XP055523802, NL ISSN: 1387-3806, DOI: 10.1016/j.ijms.2015.08.009
- Hidenori Takahashi ET AL: "Structural Analysis of Phospholipid Using Hydrogen Abstraction Dissociation and Oxygen Attachment Dissociation in Tandem Mass Spectrometry", Analytical Chemistry, vol. 90, no. 12, 24 May 2018 (2018-05-24), pages 7230-7238, XP055547319, ISSN: 0003-2700, DOI: 10.1021/acs.analchem.8b00322

## Description

### TECHNICAL FIELD

The present invention relates to a lipid-analyzing method using mass spectrometry, and a mass spectrometer for the same method.

### BACKGROUND ART

Biological activity of a lipid in a living organism significantly depends on the position of the double bond site (i.e. unsaturated bond site) of an unsaturated fatty acid. Accordingly, in a lipid analysis using tandem mass spectrometry (MS/MS), it is essential to locate the unsaturated bond site. As regards a commonly used tandem mass spectrometry method which employs low-energy collision-induced dissociation (LE-CID) for ion dissociation, it has been known that product-ion peaks originating from a fatty acid cannot be sufficiently observed, and therefore, it is difficult locate the unsaturated bond site. For this problem, the following techniques have conventionally been known as techniques for locating the unsaturated bond site.

Non Patent Literature 1 discloses the idea of ionizing a sample of lipid origin by electron ionization (EI) after derivatizing the sample with pyrrolidide or a similar substance. This technique allows for the observation of a peak pair of the product ions originating from the aliphatic chain having a mass difference of 12 Da which characteristically indicates the unsaturated bond site.

Patent Literature 1 discloses a method for locating the unsaturated bond site by utilizing the characteristic fact that the dissociation of a lipid specifically occurs at the unsaturated bond site of a fatty acid if ions derived from the lipid are made to react with ozone by introducing ozone into an ion trap in which those ions have been captured.

Patent Literature 2 and Non Patent Literature 2 disclose a method for locating the unsaturated bond site by utilizing the characteristic fact that the product ion of the unsaturated bond site has a comparatively low signal intensity among the group of product ions which are obtained by an ion dissociation method that employs high-energy electron irradiation or high-energy collision-induced dissociation.

Non Patent Literature 3 discloses a method in which helium molecules accelerated to high speeds are injected into an ion trap to turn lipid-derived ions captured within the ion trap into radical species. Those radical species are subsequently dissociated by collision-induced dissociation, whereby the product ions of the aliphatic chain are abundantly generated. Based on the signal intensities of those product ions, the unsaturated bond site is located.

The method disclosed in Non Patent Literature 1 requires a cumbersome pretreatment for derivatizing the sample. Furthermore, the analysis cannot be performed for samples that cannot be derivatized.

The method disclosed in Patent Literature 1 requires an ozone filter or similar equipment for preventing the highly reactive ozone from being released to the atmosphere. This increases the cost of the device.

In any of the methods disclosed in Patent Literature 2 as well as Non Patent Literatures 2 and 3, the phenomenon that the product ion of the unsaturated bond site has a lower signal intensity than those of the other sites is utilized to estimate the position of the unsaturated bond site. However, it is difficult to perform such an analysis in a stable manner, since there is the case where the product ions of the sites other than the unsaturated bond site have lower signal intensities, depending on the kind of sample, measurement conditions and other factors.

Thus, any of the methods mentioned so far has a defect in locating the unsaturated bond site.

LIANG ET AL: "Electron Transfer Dissociation of Doubly Sodiated Glycerophosphocholine Lipids", JOURNAL OF THE AMERICAN SOCIETY FOR MASS SPECTROMETRY, ELSEVIER SCIENCE INC, US, vol. 18, no. 10, 1 October 2007 (2007-10-01 ), pages 1783-1788, XP022262070, ISSN: 1044-0305, DOI: 10.1016/J.JASMS.2007.07.013, discloses electron transfer dissociation of doubly sodiated glycerophosphocholine lipids.

US2009/152458A1 discloses a method for fragmentation of analyte ions for mass spectroscopy and a system for mass spectroscopy. The method produces gas-phase analyte ions, produces gas-phase radical species separately from the analyte ions, and mixes the gas-phase analyte ions and the radical species at substantially atmospheric pressure conditions to produce fragment ions prior to introduction into a mass spectrometer. The system includes a gas-phase analyte ion source, a gas-phase radical species source separate from the gas-phase analyte ion source, a mixing region where the gas-phase analyte ions and the radical species are mixed at substantially atmospheric pressure to produce fragment ions of the analyte ions, a mass spectrometer having an entrance where at least a portion of the fragment ions are introduced into a vacuum of the mass spectrometer, and a detector in the mass spectrometer which determines a mass to charge ratio analysis of the fragment ions.

LI PENGFEI ET AL: "Multistage mass spectrometry of phospholipids using collision-induced dissociation (CID) and metastable atom-activated dissociation (MAD)", INTERNATIONAL JOURNAL OF MASS SPECTROMETRY, ELSEVIER SCIENCE PUBLISHERS, AMSTERDAM, NL, vol. 403, 17 March 2016 (2016-03-17), pages 1-7, XP029622070, ISSN: 1387-3806, DOI: 10.1016/ J.IJMS.2016.02.010, discloses multistage mass spectrometry of phospholipids using collision-induced dissociation and metastable atom-activated dissociation.

### CITATION LIST

### PATENT LITERATURE

Patent Literature 1: AU 2007211893 A
Patent Literature 2: CA 2951762 A
Patent Literature 3: WO 2015/133259 A

### NON PATENT LITERATURE

Non Patent Literature 1: Bengt A. Andersson and another author, "Pyrrolidides for mass spectrometric determination of the position of the double bond in monounsaturated fatty acids", Lipids, March 1974, Vol. 9, Issue 3, pp. 185-190
Non Patent Literature 2: Shimma Shuichi and three other authors, "Detailed Structural Analysis of Lipids Directly on Tissue Specimens Using a MALDI-SpiralTOF-Reflectron TOF Mass Spectrometer", PLoS One, 7, May 2012, pp. e37107
Non Patent Literature 3: Robert E. Deimler and two other authors, "Radical-induced fragmentation of phospholipid cations using metastable atom-activated dissociation mass spectrometry (MAD-MS)", International Journal of Mass Spectrometry, November 2015, Vol. 390, pp. 178-186

### SUMMARY OF INVENTION

### TECHNICAL PROBLEM

The present invention has been developed to solve the previously described problem. Its primary objective is to provide a lipid-analyzing method using mass spectrometry which does not require a cumbersome sample pretreatment, such as the derivatization, and one which can determine the position of an unsaturated bond site in a lipid in a stable manner, as well as a mass spectrometer suitable for such an analysis.

### SOLUTION TO PROBLEM

The present inventors have developed a novel ion-dissociation method called the "hydrogen-attachment dissociation", as proposed in Patent Literature 3 and other documents. Simply put, the hydrogen-attachment dissociation is a method in which hydrogen radicals are attached to ions to dissociate the ions. Using this method and its improved versions, the present inventors have conducted research mainly focused on applications of the method to an analysis of various compounds derived from living organisms. In the research process, they have found the fact that a peak pair characteristic of the unsaturated bond site in lipids can be observed with sufficient signal intensities in the product-ion spectrum obtained by appropriately combining the irradiation of the sample-derived ions with hydrogen radicals and the precursor-ion isolation or dissociating operation on those ions. Based on this finding, the present invention has been completed.

According to a first aspect of the present invention there is provided a mass spectrometer as specified in claim 1.

According to a second aspect of the present invention there is provided a lipid-analyzing method as specified in claim 11.

In the lipid-analyzing method and the mass spectrometer according to the present invention, the "radical species of a predetermined molecule" includes at least one kind of radical species selected from the hydrogen radical, hydroxyl radical, and oxygen radical.

As described in Patent Literature 3 and other documents, when the molecular ion of a component in a sample is irradiated with hydrogen radicals under appropriate conditions, the hydrogen attaches to the ion occurs. This reaction turns the ion into an active radical ion, whereby the dissociation of the ion is promoted. This is a type of radical-induced dissociation similar to the electron transfer dissociation (ETD) or electron capture dissociation (ECD). However, simply irradiating the molecular ions of a component in a sample with hydrogen radicals causes only a portion of those ions to be dissociated; a considerable number of ions remain undissociated after they have turned into radical ions. Accordingly, even in the device disclosed in Patent Literature 3, an additional process for promoting the dissociation of the ions is adopted, such as oscillating the ions to make them collide with gas particles or irradiating the ions with laser light after irradiating the ions with hydrogen radicals within an ion trap.

Such a process certainly promotes the dissociation of the ions. However, the ions (product ions) which have already undergone dissociation by the irradiation with hydrogen radicals or the like tend to be dissociated additionally, i.e. in multiple stages, in the subsequent process, such as the collision-induced dissociation. There are also radical ions which react with hydrogen radicals two or more times. The peaks of such product ions which have undergone the dissociation or reaction in multiple stages also appear in the mass spectrum (product-ion spectrum), causing the peak pattern to be complex and difficult to analyze.

In view of this problem, in the mass spectrometer according to the present invention, the operation of selectively isolating an ion having a specific mass-to-charge ratio originating from the target component as the precursor ion in the precursor-ion isolator is performed after the molecular ions of a component in a sample are irradiated with radical species of a predetermined molecule in the ion reactor to induce a reaction between the ions and the radical species. In other words, the precursor ion is isolated in the stage of the radical ions (or activated ions) generated by the reaction. Product ions which have already been generated due to the dissociation by the irradiation with the radical species are removed in this stage. Only the precursor ion having the specific mass-to-charge ratio is subsequently dissociated in the ion dissociator, and product ions are thereby generated.

In order to prevent ions from being instantly dissociated due to the irradiation with the radical species, the temperature of the ion reactor should be low. On the other hand, in order to improve the efficiency of the dissociation of the radical ions generated by the reaction with the radical species, the temperature should be high. Accordingly, in the mass spectrometer according to the present invention, the ion dissociator may include a heater for heating an area in which ions are irradiated with the radical species.

In the mass spectrometer according to the present invention, the ion dissociator may be configured to promote dissociation of the precursor ion by making a photon or neutral particle collide with the precursor ion. More specifically, the radical-induced dissociation of the precursor ion in the activated state can be promoted by irradiating the precursor ion with laser light or making the precursor ion collide with inert gas, such as argon.

The product ions generated in this manner are separated from each other according to their mass-to-charge ratios and detected in the separating-detecting section. Based on the thereby obtained detection signals, a product-ion spectrum for the ions derived from the target component can be created. As described earlier, ions which have been dissociated due to the irradiation with the radical species are removed in the stage of the precursor-ion isolation. Accordingly, the peaks which appear in the product-ion spectrum are the peaks of the product ions generated by the radical-induced dissociation in the ion dissociator. Peaks of the product ions which have undergone dissociation or reaction in multiple stages are rarely observed. Thus, a mass spectrum which allows for an easy analysis of the structure of the sample component can be obtained.

In the lipid-analyzing method according to the present invention, a mass spectrum obtained in the previously described manner is searched for a peak pair having a mass difference characteristic of the unsaturated bond site of the aliphatic chain. Typically, a peak pair having a mass difference of 12 Da may be searched for, since the mass difference characteristic of the unsaturated bond site is 12 Da. Based on the located peak pair, the position of the unsaturated bond site in the lipid is determined. Such a series of analyzing tasks may be manually performed by a user, although it is preferable that the analysis be automatically performed.

Accordingly, as one mode of the mass spectrometer according to the present invention, when the mass spectrometer is configured to perform a measurement for a lipid as a target sample, the mass spectrometer may further include:
a data analyzer for determining the position of the unsaturated bond site in the lipid based on a peak pair located by searching a mass spectrum created based on detection signals obtained by the separating-detecting section for a peak pair having a mass difference of 12 Da which characteristically appears at an unsaturated bond site of an aliphatic chain.

The mass difference of a pair of peaks which characteristically appear at the saturated bond site of the aliphatic chain is 14 Da. Accordingly, in the previously described mode of the mass spectrometer, the data analyzer may be configured to estimate the structure of the aliphatic chain based on a peak pair located by searching the mass spectrum for a peak pair having a mass difference of 14 Da which characteristically appears at the saturated bond site of the aliphatic chain.

In the mass spectrum obtained in the previously described manner, the signal intensity of a product-ion peak originating from a fatty acid bonded at the sn-2 position of glycerol is higher than that of a product-ion peak originating from a fatty acid bonded at the sn-1 position of glycerol. Accordingly, in the previously described mode of the mass spectrometer, the data analyzer may be configured to estimate a fatty acid bonded at the sn-2 position of glycerol based on the signal intensity of a product-ion peak originating from a fatty acid.

In the mass spectrum obtained in the previously described manner, the signal intensity of a product-ion peak of the saturated bond site is normally higher than that of a product-ion peak of the unsaturated bond site.

Accordingly, as another mode of the mass spectrometer according to the present invention, when the mass spectrometer is configured to perform a measurement for a lipid as a target sample, the mass spectrometer may further include a data analyzer for estimating a saturated bond site and an unsaturated bond site based on the signal intensities of product-ion peaks on a mass spectrum created based on detection signals obtained by the separating-detecting section.

Ions can also be dissociated by irradiation with electrons having a high energy of approximately 50 eV or higher. When such a method is used to dissociate lipid-derived ions, hydrogen rearrangement occurs, as noted earlier, so that the mass difference of the peak pair at the unsaturated bond site will not be 12 Da but will be increased by +2 Da. Therefore, it is impossible to locate the unsaturated bond site using the mass difference of a peak pair on a mass spectrum obtained by a method which employs electron irradiation to dissociate ions. However, the accuracy of the estimation of the unsaturated bond site can be enhanced by checking that a peak pair which corresponds to the peak pair having a mass difference of 12 Da is shifted by +2 Da on the mass spectrum obtained by a process employing the electron irradiation.

Accordingly, as still another mode of the mass spectrometer according to the present invention, when the mass spectrometer is configured to perform a measurement for a lipid as a target sample, the mass spectrometer may further include:
an electron-using ion dissociator for promoting dissociation of a precursor ion by irradiating the precursor ion with electrons, the precursor ion isolated by the precursor ion isolator without irradiating the ions with the radical species; and
a data analyzer for estimating an unsaturated bond site based on a shift in mass value of a peak originating from the same product ion on a mass spectrum obtained when the precursor ion is dissociated by the electron-using ion dissociator and a mass spectrum obtained when the precursor ion is isolated and dissociated after the reaction with the radical species.

As one mode of the mass spectrometer according to the present invention, the following operations may be carried out within an inner space of an ion trap: the irradiation of the ions with the radical species in the ion reactor; the isolation of the precursor ion in the precursor-ion isolator; and the dissociation of the precursor ion in the ion dissociator.

In this case, the ion trap may be a three-dimensional quadrupole ion trap including one ring electrode and a pair of endcap electrodes, or a quadrupole linear ion trap including four rod electrodes. The separation of ions according to their mass-to-charge ratios in the separating-detecting section may be performed with the ion trap or with another device placed outside the ion trap, such as a time-of-flight mass separator.

With this mode of the mass spectrometer, the irradiation of the ions with the radical species can be performed while the ions are captured within the ion trap. Therefore, it is easy to control the irradiation time and other conditions. This allows the reaction with the radical species to be performed in a stable manner.

As another mode of the mass spectrometer according to the present invention, the following chambers may be provided separately from each other: a reaction chamber in which the irradiation of the ions with the radical species in the ion reactor is carried out; a mass separator in which the isolation of the precursor ion in the precursor ion isolator is carried out; and a dissociation chamber in which the dissociation of the precursor ion in the ion dissociator is carried out.

One example of the mass separator in which the isolation of the precursor ion is carried out is a quadrupole mass filter. This mode of mass spectrometer may be configured as a triple quadrupole mass spectrometer or quadrupole time-of-flight mass spectrometer (q-TOF mass spectrometer) to which the reaction chamber is added. This mode of mass spectrometer can perform a series of operations on ions while transporting the ions through the reaction chamber, mass separator, dissociation chamber and separating-detecting section in the mentioned order. Such a configuration is convenient in the case where the components in a sample which is continuously introduced into the mass spectrometer need to be analyzed, as in the case of a gas chromatograph mass spectrometer or liquid chromatograph mass spectrometer.

### ADVANTAGEOUS EFFECTS OF THE INVENTION

With the lipid-analyzing method using mass spectrometry according to the present invention, a stable and accurate determination of the position of the unsaturated bond site in a lipid can be achieved without requiring a sample pretreatment, such as the derivatization, and without using ozone or other harmful substances to humans. With the mass spectrometer according to the present invention, for example, a mass spectrum which is useful for easily determining the position of the unsaturated bond site in a lipid can be assuredly obtained. Needless to say, the mass spectrometer according to the present invention is not only available for the structural analysis of lipids but is also useful for the structural analysis of various other compounds.

### BRIEF DESCRIPTION OF DRAWINGS

Fig. 1 is a schematic configuration diagram of one embodiment of the mass spectrometer according to the present invention.
Fig. 2 is a flowchart showing the analyzing and processing steps in a lipid analysis using a mass spectrometer according to the present invention.
Figs. 3A and 3B show the result of a measurement of a mass spectrum over a range including a precursor ion for lipid PC(18:1(9Z)), the mass spectrum measured before the precursor ion which had been isolated was irradiated with hydrogen radicals as well as after the irradiation with hydrogen radicals was performed.
Fig. 4 shows the result of a measurement of a product-ion spectrum obtained by a process in which the precursor ion after the irradiation with hydrogen radicals shown in Fig. 3B was dissociated by the low-energy collision-induced dissociation (LE-CID) method.
Fig. 5 shows the result of a measurement of a product-ion spectrum obtained for lipid PC(18:1(9Z)) by a process in which the precursor-ion isolation process was performed after the irradiation with hydrogen radicals, and the precursor ion was subsequently dissociated by the LE-CID method.
Fig. 6 shows the result of a measurement of a product-ion spectrum obtained lipid PC(18:1(9Z)/18:0) by a process in which the precursor-ion isolation process was performed after the irradiation with hydrogen radicals, and the precursor ion was subsequently dissociated by the LE-CID method.
Fig. 7 shows the result of a measurement of a product-ion spectrum obtained by a process in which a precursor ion derived from lipid PC(18:1(9Z)) was dissociated by the LE-CID method.
Fig. 8A show the result of a measurement of a product-ion spectrum obtained for lipid PC(18:1(9Z)/18:0) in the case where the ion dissociation method according to the present invention was used (the same as in Fig. 6), and Fig. 8B shows the result in the case where the dissociation of the ions was performed by the EID method.
Fig. 9 is a schematic configuration diagram of another embodiment of the mass spectrometer according to the present invention.

### DESCRIPTION OF EMBODIMENTS

A mass spectrometer according to the present invention, and a lipid-analyzing method using the same mass spectrometer, will be hereinafter described in detail with reference to the attached drawings.

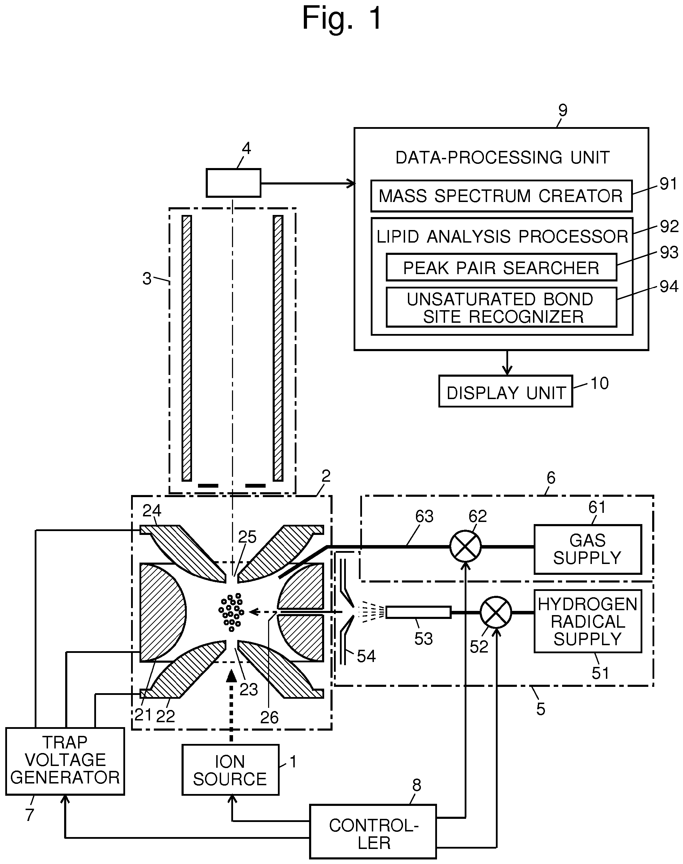

Fig. 1 is a schematic configuration diagram of a mass spectrometer system as one embodiment of the present invention.

The measurement section of the mass spectrometer system in the present embodiment includes an ion trap time-of-flight mass spectrometer. The measurement section includes the following components within a vacuum chamber (not shown) maintained in a vacuum state: an ion source 1 for ionizing components in a sample; an ion trap 2 for capturing the ions generated by the ion source 1, by the effect of a radio-frequency electric field; a time-of-flight mass separator 3 for separating ions ejected from the ion trap 2 according to their mass-to-charge ratios; and a detector 4 for detecting the separated ions. The mass spectrometer system in the present embodiment further includes: a hydrogen radical irradiator 5 for introducing hydrogen radicals into the ion trap 2; a gas supplier 6 for supplying a predetermined kind of gas into the ion trap 2; a trap voltage generator 7 for applying voltages to the electrodes constituting the ion trap 2; a controller 8 for controlling the operation of each section; a data-processing unit 9 for processing data as will be described later; and a display unit 10 for showing analysis results and other items of information.

The ion source 1 in the present embodiment is a MALDI ion source. Though not shown, the ion source 1 irradiates a sample with pulsed laser light to ionize components in the sample. The ion trap 2 is a three-dimensional quadrupole ion trap including a ring electrode 21 having a substantially annular shape as well as a pair of endcap electrodes 22 and 24 facing each other across the ring electrode 21. A linear ion trap may also be used as the ion trap 2. According to a control by the controller 8, the trap voltage generator 7 applies either a radio-frequency voltage or direct voltage, or a composite voltage of the two aforementioned voltages, to each of the ring electrode 21 as well as the endcap electrodes 22 and 24 at a predetermined timing. The time-of-flight mass separator 3 in the present example is a linear type. A different type of time-of-flight mass separator may be used, such as a reflectron or multiturn type. The use of a time-of-flight mass separator is not essential. For example, the ion-separating capability of the ion trap 2 itself may be used for mass separation. An orbitrap may also be used.

The hydrogen radical irradiator 5 includes: a hydrogel radical supply source 51 which holds or generates hydrogen radicals; a valve 52 with an adjustable flow rate; a nozzle 53 for ejecting hydrogen radicals; and a skimmer 54 having an opening on the central axis of the stream ejected from the nozzle 53, for extracting a thin stream of hydrogen radicals by separating diffused hydrogen molecules and other gas particles. The gas supplier 6 includes: a gas supply source 61 which holds helium, argon or similar gas to be used as buffer gas or cooling gas; a valve 62 with an adjustable flow rate; and a gas introduction tube 63 for transporting the gas.

The data-processing unit 9 includes, as its functional blocks, a mass spectrum creator 91 and a lipid analysis processor 92, with the latter processor including a peak pair searcher 93, unsaturated bond site recognizer 94, and other components. In normal cases, the data-processing unit 9 is actually a personal computer or workstation, with the functions of the aforementioned functional blocks realized by executing, on the computer, a dedicated processing software program installed on the same computer.

The basic measurement operation in the mass spectrometer system according to the present embodiment is hereinafter briefly described.

Various ions generated from a sample in the ion source 1 are ejected from the ion source 1 in a packet-like form. Those ions are introduced into the ion trap 2 through an ion introduction hole 23 formed in the endcap electrode 22. The ions derived from the sample component and introduced into the ion trap 2 are temporarily captured by a radio-frequency electric field created within the ion trap 2 by a radio-frequency voltage applied from the trap voltage generator 7 to the ring electrode 21. Subsequently, the operations of isolating an ion having a specific mass-to-charge ratio and dissociating the isolated ion are performed within the ion trap 2, as will be described later. Consequently, product ions originating from the target component in the sample are accumulated within the ion trap 2.

Subsequently, a high predetermined level of direct voltage is applied from the trap voltage generator 7 to the endcap electrodes 22 and 24 at a predetermined timing, whereby acceleration energy is imparted to the product ions captured within the ion trap 2, and those ions are simultaneously ejected through an ion ejection hole 25. Thus, the product ions having a specific amount of acceleration energy are introduced into the flight space of the time-of-flight mass separator 3. While flying in the flight space, the ions are separated from each other according to their mass-to-charge ratios. The detector 4 sequentially detects the separated product ions and sends detection signals to the data-processing unit 9. In this unit, the mass spectrum creator 91 creates a time-of-flight spectrum in which, for example, the point of ejection of the ions from the ion trap 2 is defined as the time of zero. Then, using mass calibration information determined beforehand, the spectrum creator 91 converts the time-of-flight into mass-to-charge ratio to create a mass spectrum formed by the product ions. In the case where the sample under analysis is a lipid, the lipid analysis processor 92 performs a predetermined data-processing operation based on the information obtained from the mass spectrum (mass information) and other related data to analyze the structure of the lipid in the sample. The analysis result is shown on the display unit 10.

The mass spectrometer system in the present embodiment is useful for the structural analysis of lipids, and particularly, for a structural analysis including the determination of the position of the unsaturated bond site. A description concerning the method for the structural analysis of a lipid in the mass spectrometer system according to the present embodiment is hereinafter given with reference to measured results, followed by the description of a specific procedure of the structural analysis.

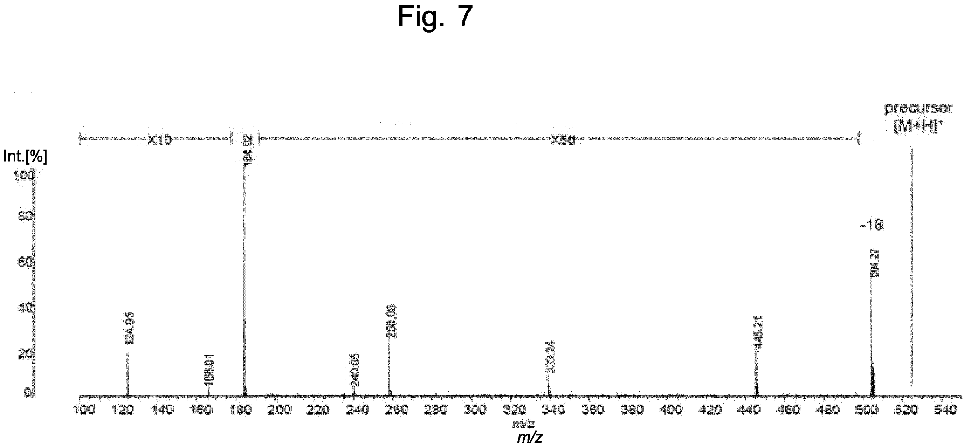

Fig. 7 shows a measured example of a mass spectrum (product-ion spectrum) obtained by using phosphatidylcholine PC(18:1(9Z)), which is a lipid with a fatty acid bonded at only one location, as the sample and dissociating a precursor ion derived from this lipid by the low-energy collision-induced dissociation (LE-CID) method, which is a conventionally and commonly used method. In the figure, product-ion peaks originating from the aliphatic chain are barely observed. Therefore, it is practically impossible to analyze the structure of the lipid from this mass spectrum.

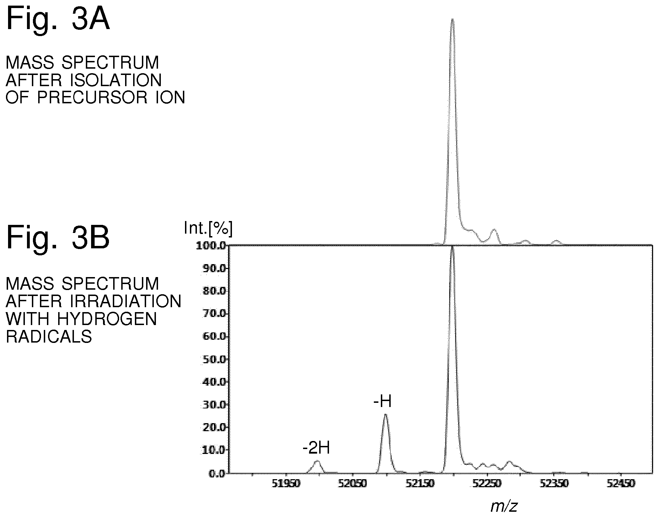

Figs. 3A and 3B show mass spectra over a range including the mass-to-charge ratio of the molecular ion derived from the phosphatidylcholine PC(18:1(9Z)) and isolated as a precursor ion. The mass spectra were measured before and after the precursor ion isolated within the ion trap 2 was irradiated with the stream of hydrogen radicals supplied from the hydrogen radical irradiator 5 into the ion trap 2. The time of irradiation with the hydrogen radicals was 500 msec. Figs. 3A and 3B demonstrate that the mass values of some of the precursor ions were shifted by 1 Da, which corresponds to the extraction of one hydrogen atom, as a result of the irradiation with the hydrogen radicals.

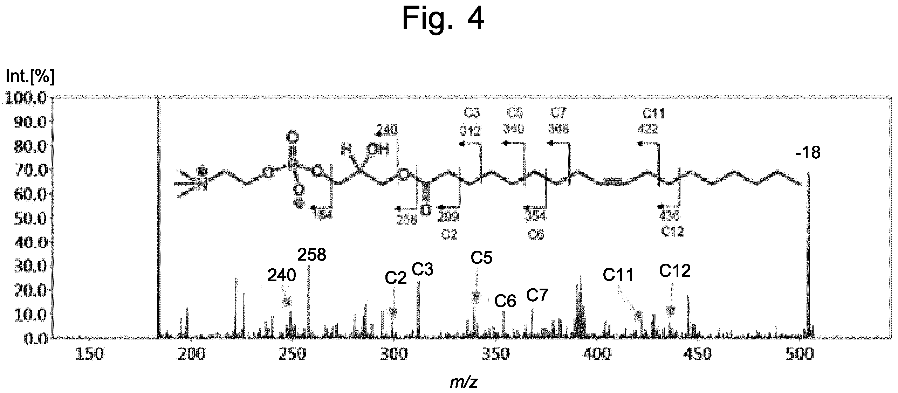

Fig. 4 shows the result of an actual measurement of a product-ion spectrum obtained by a process in which the precursor ion after the irradiation with the hydrogen radicals shown in Fig. 3B was dissociated by the LE-CID method. A comparison of Fig. 4 with Fig. 7 demonstrates that a plurality of product-ion peaks originating from the fatty acid, which were not observed in the mass spectrum obtained by the simple conventional CID method, have been observed. Since no product-ion peak is present at the unsaturated bond site of the fatty acid, it seems possible to locate the unsaturated bond site. However, there are a large number of product-ion peaks whose assignment cannot be determined. Furthermore, there are product-ion peaks whose mass values are shifted by ±1 Da due to the hydrogen rearrangement. Therefore, it is difficult to perform the structural analysis of the lipid with a sufficient level of accuracy based on this mass spectrum.

As described in Patent Literature 3 and other documents, when ions are irradiated with hydrogen radicals under appropriate conditions, dissociation of those ions occurs. The dissociation efficiency can be further improved by combining the irradiation with hydrogen radicals and LE-CID. However, in some cases, ions generated through the dissociation by the irradiation with hydrogen radicals may undergo additional dissociation due to a collision with the inert gas introduced during the LE-CID process, or re-attachment or re-desorption of hydrogen radicals to or from those ions may occur. Therefore, it is expected that some peaks whose assignment cannot be determined will appear in the mass spectrum. In view of this problem, the present inventors have conceived the method in which an ion which has been activated (but has not been dissociated yet) is isolated as the precursor ion by a precursor-ion selection process after the ions derived from the lipid have been activated by the irradiation with the hydrogen radicals, and the isolated ion is subsequently dissociated by LE-CID.

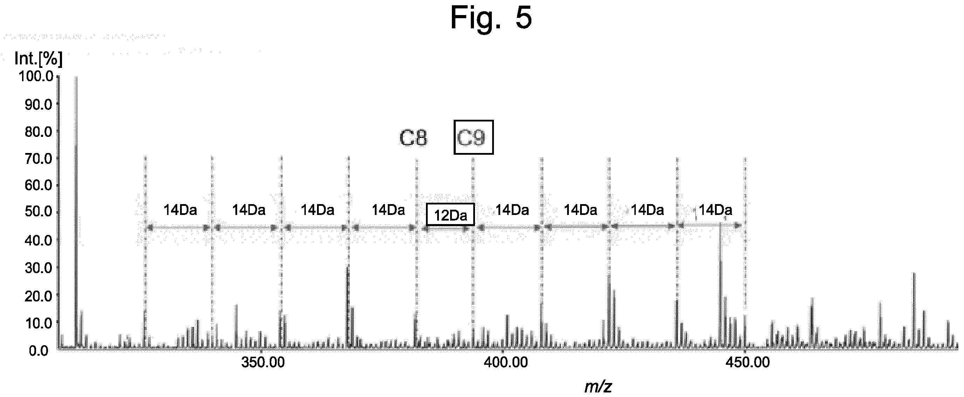

Fig. 5 shows a measured example of a product-ion spectrum obtained by a process in which the precursor-ion isolation process was performed after the molecular ion derived from PC(18:1(9Z)) was irradiated with hydrogen radicals, and the precursor ion was subsequently dissociated by LE-CID. As is evident from Fig. 5, a peak pair with a mass difference of +14 Da, which indicates the product-ion peaks of the aliphatic chain, is clearly observed in this mass spectrum. Furthermore, a peak pair with a mass difference of +12 Da, which is characteristic of the unsaturated bond site, has been observed at the unsaturated bond site (C9) of this unsaturated fatty acid. Normally, dissociating an ion by irradiation with high-energy electrons easily causes hydrogen rearrangement in the molecule. Therefore, in order to obtain a product-ion spectrum in which a peak pair with a mass difference of +12 Da characteristic of the unsaturated bond site can be detected, it is necessary to perform derivatization as disclosed in Non Patent Literature 1. By comparison, with the dissociation technique shown in Fig. 5, in which the irradiation with the hydrogen radicals, isolation of the precursor ion, and dissociation of the ions by LE-CID are successively performed, the hydrogen rearrangement can be sufficiently prevented. Therefore, it is easy to determine the assignment of each ion peak on the product-ion spectrum.

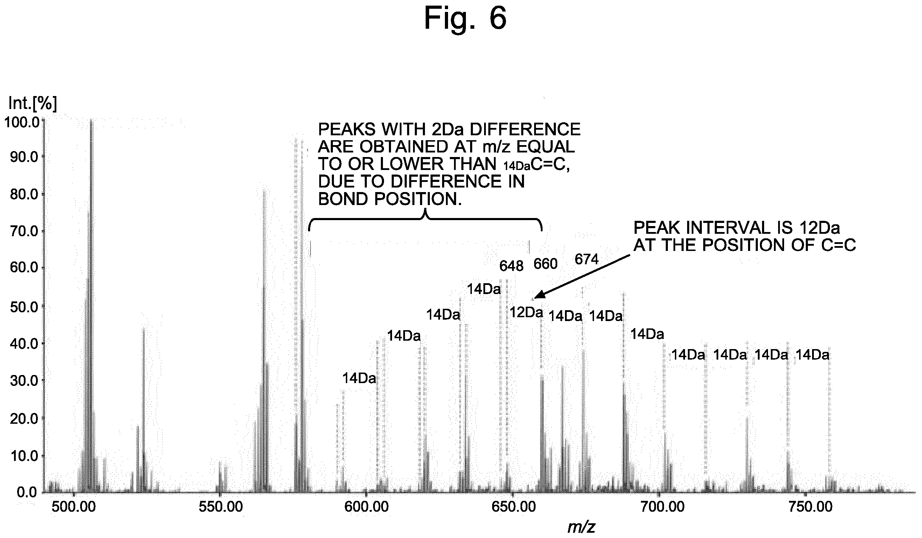

Fig. 6 shows a measured example of a product-ion spectrum obtained by a process in which the precursor-ion isolation process was performed after the molecular ion derived from phosphatidylcholine PC(18:1(9Z)/18:0), which is a lipid with two fatty acids bonded at two locations, was irradiated with hydrogen radicals, and the precursor ion was subsequently dissociated by LE-CID. Similar to the measured result shown in Fig. 5, a peak pair having a mass difference of +12 Da (648 Da-660 Da), which is characteristically observed at the unsaturated bond site, has been obtained. Peak pairs with a mass difference of +2 Da, which corresponds to the mass difference between the product-ion peak of the unsaturated aliphatic chain and that of the saturated aliphatic chain, have also been obtained within a mass-to-charge-ratio range equal to or lower than 648 Da. These results demonstrate that the position of the unsaturated bond site in PC(18:1(9Z)/18:0) can also be identified by searching for a peak pair having a mass difference of +12 Da.

As for the dissociation using the CID method, it is commonly known that a product-ion peak originating from a fatty acid bonded at the sn-2 position is observed with a relatively high intensity. This can also be confirmed in the previously described technique according to the present invention; i.e. the signal intensity of each product-ion peak originating from the sn-2 position (648-n×14 Da) is higher than that of the product-ion peak originating from the sn-1 position (646-n×14 Da). Based on this characteristic fact, it is possible to determine that the unsaturated aliphatic chain is bonded at the sn-2 position, while the saturated aliphatic chain is bonded at the sn-1 position.

In any of the examples of Figs. 5 and 6, a characteristic peak is additionally obtained at a position of +7 Da from the unsaturated bond site. By checking the presence of this peak at +7 Da along with the peak pair having a mass difference of +12 Da mentioned earlier, the accuracy of the determination of the position of the unsaturated bond site can be enhanced. However, it should be noted that, although hydrogen rearrangement does not easily occur, it is still possible that that the hydrogen rearrangement occurs. Therefore, it is preferable to allow for an occurrence of the hydrogen rearrangement and search for the ion peak not only at +7 Da from the unsaturated bond site but also within a range of +6 to +8 Da.

Furthermore, as noted earlier, the signal intensity of the product-ion peak of the unsaturated bond site is normally lower than that of the product-ion peak of the saturated bond site. Accordingly, it is preferable to utilize the signal intensities of the product-ion peaks for the estimation of the unsaturated bond site and the saturated bond site in addition to the result of the search for the peak pair mentioned earlier.

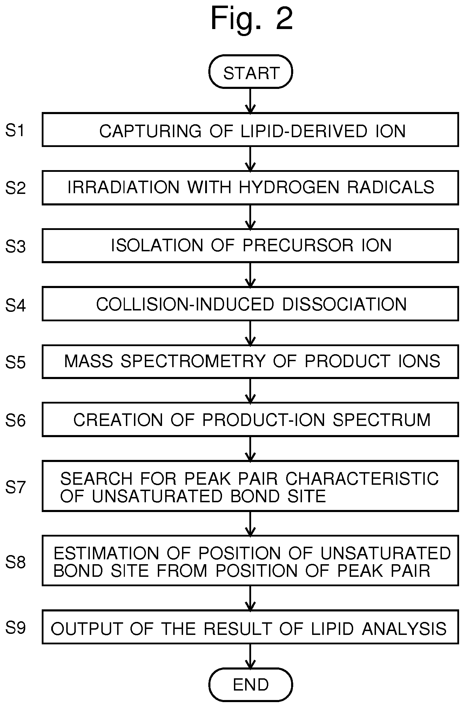

A specific procedure of the structural analysis of a lipid in the mass spectrometer system according to the present embodiment is hereinafter described with reference to the flowchart shown in Fig. 2.

Initially, ions generated from a lipid sample in the ion source 1 are captured within the ion trap 2 (Step S 1). A stream of hydrogen radicals is subsequently supplied from the hydrogen radical irradiator 5 into the ion trap 2 through a radical particle introduction port 26 formed in the ring electrode 21, to irradiate the lipid-derived ions with hydrogen radicals (Step S2). The irradiation with the hydrogen radicals causes the reaction of hydrogen extraction from the lipid-derived ions, whereby the ions are turned into radical ions. In this process, some of the ions undergo dissociation. Before the irradiation with the hydrogen radicals, a preliminary precursor-ion isolation process may be performed by applying predetermined voltages from the trap voltage generator 7 to the ring electrode 21 and other elements to remove ions other than those derived from the target lipid.

After the irradiation with the hydrogen radicals, a precursor-ion isolation process for removing ions other than those derived from the target lipid is performed by applying predetermined voltages from the trap voltage generator 7 to the ring electrode 21 and other elements (Step S3). The operation in Step S3 is invariably performed, regardless of whether the preliminary precursor-ion isolation process mentioned earlier is performed. By this operation, product ions generated due to the dissociation during the irradiation with hydrogen radicals are removed, and only the lipid-derived molecular ion which has turned into a radical ion is selected as the precursor ion. Subsequently, a predetermined kind of inert gas is introduced from the gas supplier 6 into the ion trap 2, and a predetermined radio-frequency voltage is applied from the trap voltage generator 7 to the ring electrode 21. The ions captured within the ion trap 2 are thereby oscillated, colliding with the gas particles. Thus, the ions are dissociated by LE-CID (Step S4). This dissociation is a type of radical-induced dissociation. Various product ions originating from the lipid are thereby generated.

In order to avoid instant dissociation of the ions due to the irradiation with the hydrogen radicals, the irradiation with the hydrogen radicals should be performed at a comparatively low temperature. On the other hand, in order to improve the ion-dissociating efficiency by LE-CID, the temperature should be high. Accordingly, the gas supplier 6 may be equipped with a heater so that the inert gas introduced into the ion trap 2 can be heated by the heater during the LE-CID process.

Subsequently, cooling gas is introduced from the gas supplier 6 into the ion trap 2. The product ions generated within the ion trap 2 are cooled by coming in contact with the cooling gas. After being sufficiently cooled, the product ions are simultaneously ejected from the ion trap 2 into the time-of-flight mass separator 3, where those ions are separated from each other according to their mass-to-charge ratios and sequentially detected by the detector 4 (Step S5). Based on the detection signals obtained with the detector 4, the mass spectrum creator 91 creates a mass spectrum (product-ion spectrum) for the target lipid (Step S6).

This mass spectrum has many peaks with various characteristics which depend on the structure of the lipid, including a peak pair having a mass difference characteristic of the unsaturated bond site. Accordingly, the peak pair searcher 93 searches for a peak pair having a mass difference of +12 Da, for example, among those peaks detected on the mass spectrum (Step S7). When such a peak pair has been located, the unsaturated bond site recognizer 94 estimates the position of the unsaturated bond site based on the position of the peak pair in the sequence of peaks corresponding to the aliphatic chain (Step S8). It is also possible to verify the result of the estimation of the unsaturated bond site, for example, by determining whether or not a peak is present within the range of +6 to +8 Da from the estimated position of the unsaturated bond site. The signal intensities of the peaks may additionally be utilized in the previously described manner to distinguish between the unsaturated bond site and the saturated bond site. The result of the structural analysis of the lipid obtained in this manner is shown on the display unit 10 (Step S9).

Thus, with the mass spectrometer system according to the present embodiment, the structural analysis of a lipid can be easily performed in a stable manner. Such an analysis has conventionally been rather cumbersome.

In the mass spectrometer system according to the previous embodiment, hydrogen radical is used for activating the lipid-derived ions. As described in the Japanese Patent Application No. 2017-74183, which is an earlier application filed by the present applicant, it has been known that hydroxyl radical or oxygen radical may be used in place of the hydrogen radical for the irradiation to similarly generate radical ions by the reaction of hydrogen extraction or oxygen attachment. Accordingly, it is possible to irradiate ions captured within the ion trap 2 with hydroxyl radicals or oxygen radicals in place of the hydrogen radicals. In the case of using hydrogen radicals, an ion generated by the addition of one oxygen atom to the precursor ion is detected at a mass of +16 Da relative to the mass of the precursor ion. Therefore, in this case, an ion having a mass of +16 Da relative to the lipid-derived ion should be isolated as the precursor ion for the LE-CID process, whereby a peak pair having a mass difference of +12 Da which characteristically indicates the unsaturated bond site can be obtained.

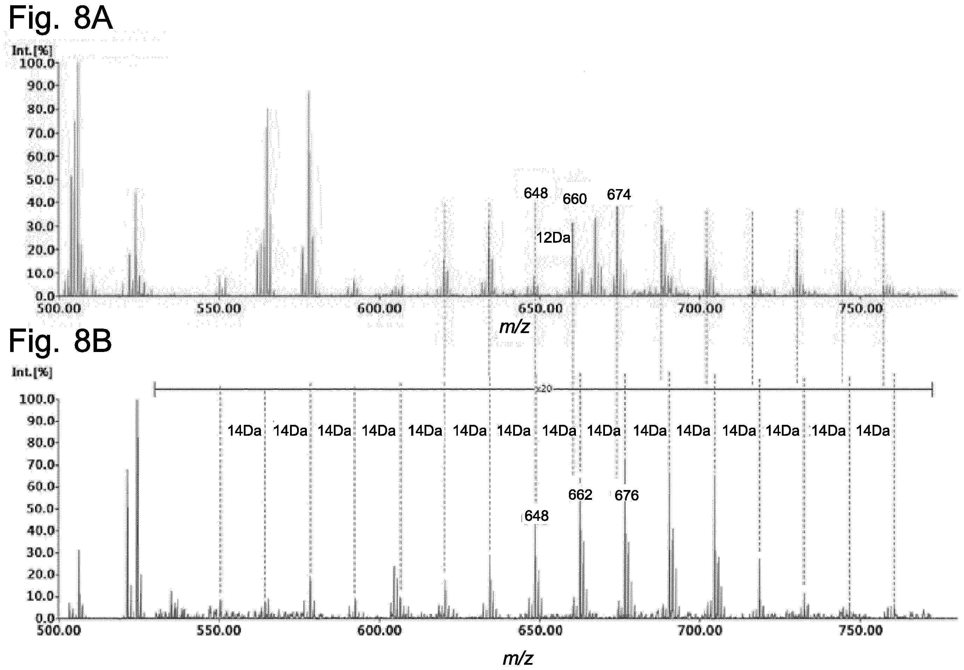

Fig. 8A shows the same product-ion spectrum as shown in Fig. 6. Fig. 8B shows the result of a measurement of a product-ion spectrum for PC(18:1(9Z)/18:0) in the case where the dissociation of the ions was performed by the electron induced dissociation (EID) method. The EID method is a technique for promoting the dissociation of ions by irradiating the ions with electrons having a high energy of approximately 50 eV or higher, as with the electron ionization or similar techniques.

In the mass spectrum shown in Fig. 8B, unlike the one shown in Fig. 8B, the characteristic peak pair having a mass difference of 12 Da is not observed at the unsaturated bond site (662 Da and 676 Da). The reason is because, as with the electron ionization, the dissociation of a lipid by the EID method causes hydrogen rearrangement, whereby the mass values of the product-ion peaks of the unsaturated bond site are shifted by +2 Da (+2H). It is difficult to estimate the unsaturated bond site from this mass spectrum unless an additional process is performed, such as the fixation of the position of the electric charges in the sample-derived ions by derivatization or other techniques. However, this mass spectrum is still useful, since the peaks whose mass values have been shifted by +2 Da can be unmistakably recognized by comparing the peaks on a mass spectrum obtained by the method according to the present invention and the peaks on a mass spectrum obtained by the method using the electron irradiation dissociation.

In the example shown in Figs. 8A and 8B, a comparison of the two mass spectra recognizably demonstrates that each peak having a mass-to-charge ratio equal to or greater than 660 Da is shifted by a mass value of +2 Da. From this fact, it is possible to recognize that 660 Da (C9) is the unsaturated bond site.

The previously described embodiment is basically premised on the case where the ion to be dissociated is the molecular ion [M+H]⁺ originating from a lipid. It is not always necessary to select the molecular ion as the precursor ion if there is a more appropriate form of ion originating from the lipid. For example, for some kinds of samples, an ion consisting of the molecule with sodium added, [M+Na]⁺, can be obtained by adding sodium acetate to the sample solvent. Such an alkali-metal adduct may also be selected as the precursor ion. As compared to the proton adduct, such an alkali-metal-adduct ion tends have the electric charge located at a fixed position in the molecule, so that the yield of the product ions originating from the radicals tends to be higher. This is useful for improving the sensitivity for detecting the ion peak on the mass spectrum.

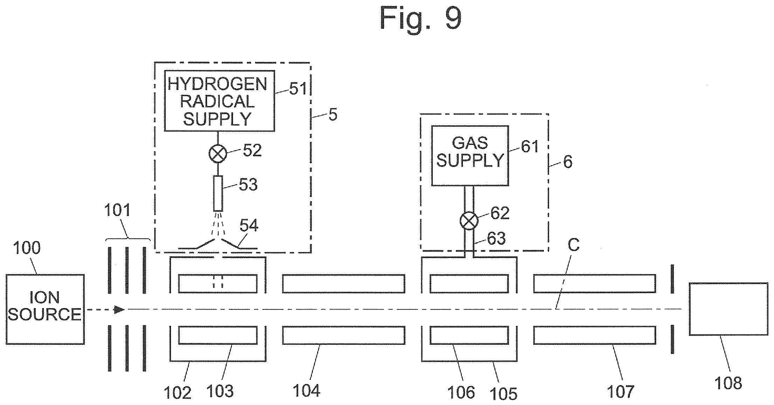

A mass spectrometer system according to another embodiment of the present invention is hereinafter described with reference to Fig. 9.

In the mass spectrometer system according to the previous embodiment shown in Fig. 1, the ion trap 2 is used as three areas: the ion reaction area in which ions are irradiated with hydrogen radicals to induce the reaction of hydrogen extraction on the ions; the ion isolation area in which the precursor-ion isolation process is performed; and the ion dissociation area in which ions are dissociated. Therefore, ions derived from the next sample to be analyzed cannot be introduced into the ion trap 2 until the ejection of ions from the ion trap 2 after the ion dissociation process is completed. This limits the possibility of enhancing of the measurement cycle. By comparison, in the mass spectrometer system shown in Fig. 9, the ion reaction area, ion isolation area, and ion dissociation area are spatially separated from each other.

More specifically, the mass spectrometer system according to the present embodiment includes the following components within a vacuum chamber (not shown): an ion source 100; an ion transport optical system 101, such as an ion lens; an ion reaction chamber 102 containing an ion guide 103; a front quadrupole mass filter 104; a collision chamber 105 containing an ion guide 106; a rear quadrupole mass filter 107; and a detector 108. The ion reaction chamber 102 and the collision chamber 105 are equipped with a hydrogen radical irradiator 5 and a gas supplier 6, respectively, which are similar to the ones included in the mass spectrometer shown in Fig. 1. The aforementioned components are arranged along a substantially straight ion beam axis C. It is not always necessary for those components to be arranged along a straight line.

If the sample is a gas sample, the ion source 100 is an ion source which employs electron ionization, chemical ionization, negative chemical ionization or other the like. If the sample is a liquid sample, the ion source 100 is an atmospheric pressure ion source which employs electrospray ionization, atmospheric pressure chemical ionization or the like. If the sample is a solid sample, an ion source employing the DART (direct analysis in real time) or the like can be used as the ion source 100.

In any case, ions generated from a sample component in the ion source 100 are introduced into the ion reaction chamber 102 and irradiated with the stream of hydrogen radicals supplied from the hydrogen radical irradiator 5. A radio-frequency voltage is applied to the ion guide 103 to generate a radio-frequency electric field, whereby the ions are made to converge into an area near the ion beam axis C. The ions derived from the sample component and activated by the irradiation with the hydrogen radicals are introduced into the front quadrupole mass filter 104. Among those ions, only an ion having a specific mass-to-charge ratio is allowed to pass through the front quadrupole mass filter 104 and enter the collision chamber 105 as the precursor ion. A predetermined kind of gas is continuously or intermittently supplied into the collision chamber 105. The ion which has entered the collision chamber 105 with a certain amount of energy collides with this gas and is dissociated by the LE-CID process.

The product ions generated by the dissociation are introduced into the rear quadrupole mass filter 107. A voltage which is controlled to sweep a specific voltage range to repeat a scan measurement over a predetermined range of mass-to-charge ratios is applied to the rod electrodes forming the rear quadrupole mass filter 107. An ion which has been allowed to pass through this rear quadrupole mass filter 107 enters the detector 108 and is thereby detected. With such a system, a product-ion spectrum can be repeatedly acquired based on the detection signals obtained with the detector 108.

This mass spectrometer system has the configuration of a triple quadrupole mass spectrometer to which the ion reaction chamber 102 is added. This system can repeatedly acquire a product-ion spectrum for the components in a sample which is almost continuously supplied to the ion source 100. Such a system is convenient for use, for example, as a gas chromatograph mass spectrometer or liquid chromatograph mass spectrometer. Needless to say, if it is preferable to increase the period of time for the irradiation with the hydrogen radicals within the ion reaction chamber 102, the ion guide 103 may be configured to function as a linear ion trap to temporarily store the ions. Additionally, the ion reaction chamber 102 and the front quadrupole mass filter 104 may be integrated into a single unit.

It is evident that the configuration shown in Fig. 9 may be changed to the q-TOF configuration which employs an orthogonal acceleration time-of-flight mass separator in place of the rear quadrupole mass filter 107.

It should be noted that the previous embodiments are mere examples of the present invention, and any change, addition or modification may be made within the scope of the appended claims.

### REFERENCE SIGNS LIST

- 1, 100: Ion Source
- 2: Ion Trap
- 21: Ring Electrode
- 22, 24: Endcap Electrode
- 23: Ion Introduction Hole
- 25: Ion Ejection Hole
- 26: Radical Particle Introduction Port
- 3: Time-of-Flight Mass Separator
- 4, 108: Detector
- 5: Hydrogen Radical Irradiator
- 51: Hydrogen Radical Supply Source
- 52: Valve
- 53: Nozzle
- 54: Skimmer
- 6: Gas Supplier
- 61: Gas Supply Source
- 62: Valve
- 63: Gas Introduction Tube
- 7: Trap Voltage Generator
- 8: Controller
- 9: Data-Processing Unit
- 91: Mass Spectrum Creator
- 92: Lipid Analysis Processor
- 93: Peak Pair Searcher
- 94: Unsaturated Bond Site Recognizer
- 10: Display Unit
- 101: Ion Transport Optical System
- 102: Ion Reaction Chamber
- 103, 106: Ion Guide
- 104.: Front Quadrupole Mass Filter
- 105: Collision Chamber
- 107: Rear Quadrupole Mass Filter
- C: Ion Beam Axis

## Claims

1. A mass spectrometer for performing mass spectrometry, comprising:
a) an ion reactor (2, 5, 102) configured to irradiate ions originating from a component in a sample with radical species of a predetermined molecule to induce a reaction between the ions and the radical species, wherein the radical species of the predetermined molecule includes at least one kind of radical species selected from hydrogen radical, hydroxyl radical, and oxygen radical, wherein irradiation with hydrogen radicals induces a reaction of hydrogen extraction, and wherein irradiation with hydroxyl radicals or oxygen radicals induces a reaction of hydrogen extraction or oxygen attachment;
b) a precursor ion isolator (2, 104) configured to selectively isolate a specific ion which has turned into a radical ion by said reaction as a precursor ion, so that product ions generated due to dissociation by irradiation of the ions with the radical species are removed;
c) an ion dissociator (2, 6, 105) configured to dissociate the precursor ion isolated by the precursor ion isolator (2, 104) to generate product ions; and
d) a separating-detecting section (3, 4, 107, 108) configured to separate the product ions generated by the ion dissociator (2, 6, 105) from each other according to their mass-to-charge ratios, and configured to detect the separated product ions.

2. The mass spectrometer according to claim 1, wherein:
the ion dissociator (2, 6, 105) promotes dissociation of the precursor ion by making a photon or neutral particle collide with the precursor ion.

3. The mass spectrometer according to claim 1, wherein:
the ion dissociator (2, 6, 105) comprises a gas supplier (6) equipped with a heater.

4. The mass spectrometer according to claim 1, wherein:
following operations are carried out within an inner space of an ion trap: irradiation of the ions with the radical species in the ion reactor (2, 5, 102); isolation of the precursor ion in the precursor ion isolator (2, 104); and dissociation of the precursor ion in the ion dissociator (2, 6, 105).

5. The mass spectrometer according to claim 1, wherein:
following chambers are provided separately from each other: a reaction chamber (102) in which irradiation of the ions with the radical species in the ion reactor (5, 102) is carried out; a mass separator (104) in which isolation of the precursor ion in the precursor ion isolator (104) is carried out; and a dissociation chamber (105) in which dissociation of the precursor ion in the ion dissociator (6, 105) is carried out.

6. The mass spectrometer according to claim 1, the mass spectrometer configured to perform a measurement for a lipid as a target sample, and the mass spectrometer further comprising:
a data analyzer (9) configured to determine a position of an unsaturated bond site in the lipid based on a peak pair located by searching a mass spectrum created based on detection signals obtained by the separating-detecting section (3, 4, 107, 108) for a peak pair having a mass difference of 12 Da which characteristically appears at an unsaturated bond site of an aliphatic chain.

7. The mass spectrometer according to claim 6, wherein:
the data analyzer (9) is configured to estimate a structure of the aliphatic chain based on a peak pair located by searching the mass spectrum for a peak pair having a mass difference of 14 Da which characteristically appears at a saturated bond site of an aliphatic chain.

8. The mass spectrometer according to claim 6, wherein:
the data analyzer (9) is configured to estimate a fatty acid bonded at an sn-2 position of glycerol based on a signal intensity of a product-ion peak originating from the aliphatic chain.

9. The mass spectrometer according to claim 1, the mass spectrometer configured to perform a measurement for a lipid as a target sample, and the mass spectrometer further comprising:
a data analyzer (9) configured to estimate a saturated bond site and an unsaturated bond site based on signal intensities of product-ion peaks on a mass spectrum created based on detection signals obtained by the separating-detecting section (3, 4, 107, 108).

10. The mass spectrometer according to claim 1, the mass spectrometer configured to perform a measurement for a lipid as a target sample, and the mass spectrometer further comprising:
an electron-using ion dissociator configured to promote dissociation of a precursor ion by irradiating the precursor ion with electrons, the precursor ion isolated by the precursor ion isolator (2, 104) without irradiating the ions with the radical species; and
a data analyzer (9) configured to estimate an unsaturated bond site based on a shift in mass value of a peak originating from the same product ion on a mass spectrum obtained when the precursor ion is dissociated by the electron-using ion dissociator and a mass spectrum obtained when the precursor ion is isolated and dissociated after the reaction with the radical species.

11. A lipid-analyzing method using mass spectrometry, comprising:
a) an ion reaction step in which ions originating from a target sample including a lipid are irradiated with radical species of a predetermined molecule to induce a reaction between the ions and the radical species, wherein the radical species of the predetermined molecule includes at least one kind of radical species selected from hydrogen radical, hydroxyl radical, and oxygen radical, wherein irradiation with hydrogen radicals induces a reaction of hydrogen extraction, and wherein irradiation with hydroxyl radicals or oxygen radicals induces a reaction of hydrogen extraction or oxygen attachment;
b) a precursor ion isolation step in which a specific ion which has turned into a radical ion by said reaction is selectively isolated as a precursor ion, so that product ions generated due to dissociation by irradiation of the ions with the radical species are removed;
c) an ion dissociation step in which the precursor ion isolated in the precursor ion isolation step is dissociated to generate product ions;
d) a separation-detection step in which the product ions generated in the ion dissociation step are separated from each other according to their mass-to-charge ratios and detected as detection signals; and
e) a data analysis step in which a mass spectrum created based on the detection signals obtained in the separation-detection step is searched for a peak pair having a mass difference characteristic of an unsaturated bond site of an aliphatic chain, and a position of the unsaturated bond site in the lipid is determined based on the located peak pair.

12. The lipid-analyzing method according to claim 11, wherein:
the data analyzing step includes searching for a peak pair having a mass difference of 12 Da.

## Patentansprüche

1. Massenspektrometer zur Ausführung von Massenspektrometrie, das Folgendes umfasst:
a) einen lonenreaktor (2, 5, 102), der dazu ausgelegt ist, Ionen zu bestrahlen, die von einer Komponente in einer Probe mit einer Radikalspezies eines vorbestimmten Moleküls stammen, um eine Reaktion zwischen den Ionen und der Radikalspezies zu induzieren, wobei die Radikalspezies des vorbestimmten Moleküls zumindest eine Art von Radikalspezies umfassen, die aus einem Wasserstoffradikal, einem Hydroxylradikal und einem Sauerstoffradikal ausgewählt ist, wobei die Bestrahlung mit Wasserstoffradikalen eine Wasserstoffextraktionsreaktion induziert und wobei die Bestrahlung mit Hydroxylradikalen oder Sauerstoffradikalen eine Wasserstoffextraktions- oder Sauerstoffanbindungsreaktion induziert;
b) einen Vorläuferionenisolator (2, 104), der dazu ausgelegt ist, ein spezifisches Ion selektiv zu isolieren, das durch die Reaktion als Vorläuferion zu einem Radikalion wurde, so dass aufgrund der Dissoziation durch Bestrahlung der Ionen mit der Radikalspezies erzeugte Produktionen entfernt werden;
c) einen lonendissoziator (2, 6, 105), der dazu ausgelegt ist, das durch den Vorläuferionenisolator (2, 104) isolierte Vorläuferion zu dissoziieren, um Produktionen zu erzeugen; und
d) einen Trenn-Detektions-Abschnitt (3, 4, 107, 108), der dazu ausgelegt ist, die durch den lonendissoziator (2, 6, 105) erzeugten Produktionen gemäß ihren Masse-Ladungs-Verhältnissen voneinander zu trennen, und dazu ausgelegt ist, die getrennten Produktionen zu detektieren.

2. Massenspektrometer nach Anspruch 1, wobei:
der lonendissoziator (2, 6, 105) die Dissoziation des Vorläuferions fördert, indem er ein Photon oder neutrales Partikel mit dem Vorläuferion zum Kollidieren bringt.

3. Massenspektrometer nach Anspruch 1, wobei:
der lonendissoziator (2, 6, 105) eine Gasversorgungsvorrichtung (6) umfasst, die mit einer Heizvorrichtung ausgestattet ist.

4. Massenspektrometer nach Anspruch 1, wobei:
die folgenden Operationen im Inneren einer lonenfalle ausgeführt werden: das Bestrahlen der Ionen mit der Radikalspezies im lonenreaktor (2, 5, 102); das Isolieren des Vorläuferions im Vorläuferionenisolator (2, 104); und das Dissoziieren des Vorläuferions im lonendissoziator (2, 6, 105).

5. Massenspektrometer nach Anspruch 1, wobei:
die folgenden Kammern getrennt voneinander bereitgestellt sind: eine Reaktionskammer (102), in der eine Bestrahlung der Ionen mit den Radikalspezies im lonenreaktor (5, 102) ausgeführt wird; einen Massenseparator (104), in dem eine Isolierung des Vorläuferions im Vorläuferionenisolator (104) ausgeführt wird; und eine Dissoziationskammer (105), in der eine Dissoziation des Vorläuferions im lonendissoziator (6, 105) ausgeführt wird.

6. Massenspektrometer nach Anspruch 1, wobei das Massenspektrometer dazu ausgelegt ist, eine Messung mit einem Lipid als Zielprobe auszuführen, und das Massenspektrometer weiters Folgendes umfasst:
einen Datenanalysator (9), der dazu ausgelegt ist, die Position einer ungesättigten Bindungsstelle im Lipid basierend auf einem Peak-Paar zu bestimmen, das durch Durchsuchen eines Massenspektrums lokalisiert wird, das basierend auf Detektionssignalen erstellt wurde, die durch den Trenn-Detektions-Abschnitt (3, 4, 107, 108) erhalten wurden, und zwar für ein Peak-Paar mit einer Massendifferenz von 12 Da, die charakteristischerweise an einer ungesättigten Bindungsstelle einer aliphatischen Kette auftritt.

7. Massenspektrometer nach Anspruch 6, wobei:
der Datenanalysator (9) dazu ausgelegt ist, die Struktur der aliphatischen Kette basierend auf einem Peak-Paar abzuschätzen, das durch Durchsuchen des Massenspektrums auf ein Peak-Paar mit einer Massendifferenz von 14 Da lokalisiert wurde, die charakteristischerweise an einer ungesättigten Bindungsstelle einer aliphatischen Kette auftritt.

8. Massenspektrometer nach Anspruch 6, wobei:
der Datenanalysator (9) dazu ausgelegt ist, eine Fettsäure, die an einer sn-2-Position von Glycerin gebunden ist, basierend auf der Signalintensität eines Produktionen-Peaks zu schätzen, der von der aliphatischen Kette stammt.

9. Massenspektrometer nach Anspruch 1, wobei das Massenspektrometer dazu ausgelegt ist, eine Messung mit einem Lipid als Zielprobe auszuführen, und das Massenspektrometer weiters Folgendes umfasst:
einen Datenanalysator (9), der dazu ausgelegt ist, eine gesättigte Bindungsstelle und eine ungesättigte Bindungsstelle basierend auf Signalintensitäten von Produktionen-Peaks in einem Massenspektrum abzuschätzen, das basierend auf Detektionssignalen erstellt wurde, die durch den Trenn-Detektions-Abschnitt (3, 4, 107, 108) erhalten wurden.

10. Massenspektrometer nach Anspruch 1, wobei das Massenspektrometer dazu ausgelegt ist, eine Messung mit einem Lipid als Zielprobe auszuführen und das Massenspektrometer weiters Folgendes umfasst:
einen Elektronen nutzenden lonendissoziator, der dazu ausgelegt ist, die Dissoziation eines Vorläuferions durch Bestrahlen des Vorläuferions mit Elektronen zu fördern, wobei das Vorläuferion durch den Vorläuferionenisolator (2, 104) ohne Bestrahlen der Ionen mit der Radikalspezies isoliert wurde; und
einen Datenanalysator (9), der dazu ausgelegt ist, eine ungesättigte Bindungsstelle basierend auf einer Verschiebung des Massenwerts eines Peaks zu bestimmen, der vom gleichen Produktion in einem Massenspektrum, das erhalten wurde, als das Vorläuferion durch den Elektronen verwendenden lonendissoziator dissoziiert wurde, und einem Massenspektrum stammt, das erhalten wurde, als das Vorläuferion nach der Reaktion mit der Radikalspezies isoliert und dissoziiert wurde.

11. Lipidanalyseverfahren unter Verwendung von Massenspektrometrie, das Folgendes umfasst:
a) einen lonenreaktionsschritt, bei dem Ionen, die aus einer Zielprobe stammen, die ein Lipid umfasst, mit einer Radikalspezies eines bestimmten Moleküls bestrahlt werden, um eine Reaktion zwischen den Ionen und der Radikalspezies zu induzieren, wobei die Radikalspezies des vorbestimmten Moleküls zumindest eine Art von Radikalspezies umfasst, die aus einem Wasserstoffradikal, einem Hydroxylradikal und einem Sauerstoffradikal ausgewählt ist, wobei die Bestrahlung mit Wasserstoffradikalen eine Wasserstoffextraktionsreaktion induziert und wobei die Bestrahlung mit Hydroxylradikalen oder Sauerstoffradikalen eine Wasserstoffextraktions- oder Sauerstoffanbindungsreaktion induziert;
b) einen Vorläuferionen-Isolierungsschritt, bei dem ein bestimmtes Ion, das durch die Reaktion zu einem Radikalion wurde, selektiv als Vorläuferion isoliert wird, so dass aufgrund der Dissoziation durch Bestrahlung der Ionen mit der Radikalspezies erzeugte Produktionen entfernt werden;
c) einen lonendissoziationsschritt, bei dem das durch den Vorläuferionen-Isolierungsschritt isolierte Vorläuferion dissoziiert wird, um Produktionen zu erzeugen; und
d) einen Trenn-Detektions-Schritt, bei dem die im lonendissoziationsschritt erzeugten Produktionen gemäß ihren Masse-Ladungs-Verhältnissen voneinander getrennt und als Detektionssignale detektiert werden; und
e) einen Datenanalyseschritt, bei dem ein Massenspektrum, das basierend auf Detektionssignalen erstellt wurde, die durch den Trenn-Detektions-Schritt erhalten wurden, auf ein Peak-Paar mit einer Massendifferenz durchsucht wird, die charakteristisch für eine ungesättigte Bindungsstelle einer aliphatischen Kette ist, und die Position der ungesättigten Bindungsstelle im Lipid basierend auf dem lokalisierten Peak-Paar bestimmt wird.

12. Lipidanalyseverfahren nach Anspruch 11, wobei:
der Datenanalyseschritt das Durchsuchen auf ein Peak-Paar mit einer Massendifferenz von 12 Ka umfasst.

## Revendications

1. Spectromètre de masse pour effectuer une spectrométrie de masse, comprenant :
a) un réacteur ionique (2, 5, 102) configuré pour irradier des ions provenant d'un composant dans un échantillon avec des espèces radicalaires d'une molécule prédéterminée pour induire une réaction entre les ions et les espèces radicalaires, dans lequel les espèces radicalaires de la molécule prédéterminée incluent au moins un type d'espèces radicalaires choisies parmi un radical hydrogène, un radical hydroxyle et un radical oxygène, dans lequel une irradiation avec des radicaux hydrogène induit une réaction d'extraction d'hydrogène, et dans lequel une irradiation avec des radicaux hydroxyle ou des radicaux oxygène induit une réaction d'extraction d'hydrogène ou de fixation d'oxygène ;
b) un isolateur d'ion précurseur (2, 104) configuré pour isoler sélectivement un ion spécifique qui s'est transformé en un ion radicalaire par ladite réaction en tant qu'ion précurseur, de sorte que des ions produits générés en raison de la dissociation par irradiation des ions avec les espèces radicalaires sont éliminés ;
c) un dissociateur d'ions (2, 6, 105) configuré pour dissocier l'ion précurseur isolé par l'isolateur d'ion précurseur (2, 104) pour générer des ions produits ; et
d) une section de détection de séparation (3, 4, 107, 108) configurée pour séparer les ions produits générés par le dissociateur d'ions (2, 6, 105) les uns des autres selon leurs rapports masse/charge, et configurée pour détecter les ions produits séparés.

2. Spectromètre de masse selon la revendication 1, dans lequel :
le dissociateur d'ions (2, 6, 105) favorise la dissociation de l'ion précurseur en faisant entrer en collision un photon ou une particule neutre avec l'ion précurseur.

3. Spectromètre de masse selon la revendication 1, dans lequel :
le dissociateur d'ions (2, 6, 105) comprend un dispositif d'alimentation en gaz (6) équipé d'un dispositif de chauffage.

4. Spectromètre de masse selon la revendication 1, dans lequel :
les opérations suivantes sont effectuées dans un espace intérieur d'un piège à ions : irradiation des ions avec les espèces radicalaires dans le réacteur à ions (2, 5, 102) ; isolation de l'ion précurseur dans l'isolateur d'ions précurseur (2, 104) ; et dissociation de l'ion précurseur dans le dissociateur d'ions (2, 6, 105).

5. Spectromètre de masse selon la revendication 1, dans lequel :
des chambres suivantes sont prévues séparément les unes des autres : une chambre de réaction (102) dans laquelle une irradiation des ions avec les espèces radicalaires dans le réacteur ionique (5, 102) est effectuée ; un séparateur de masse (104) dans lequel une isolation de l'ion précurseur dans l'isolateur d'ion précurseur (104) est effectuée ; et une chambre de dissociation (105) dans laquelle la dissociation de l'ion précurseur dans le dissociateur d'ions (6, 105) est effectuée.

6. Spectromètre de masse selon la revendication 1, le spectromètre de masse étant configuré pour effectuer une mesure d'un lipide en tant qu'échantillon cible, et le spectromètre de masse comprenant en outre :
un analyseur de données (9) configuré pour déterminer une position d'un site de liaison insaturée dans le lipide sur la base d'une paire de crêtes située en recherchant un spectre de masse créé sur la base de signaux de détection obtenus par la section de séparation-détection (3, 4, 107, 108) pour une paire de crêtes présentant une différence de masse de 12 Da qui apparaît de manière caractéristique au niveau d'un site de liaison insaturée d'une chaîne aliphatique.

7. Spectromètre de masse selon la revendication 6, dans lequel :
l'analyseur de données (9) est configuré pour estimer une structure de la chaîne aliphatique sur la base d'une paire de crêtes située en recherchant le spectre de masse pour une paire de crêtes présentant une différence de masse de 14 Da qui apparaît de manière caractéristique au niveau d'un site de liaison saturée d'une chaîne aliphatique.

8. Spectromètre de masse selon la revendication 6, dans lequel :
l'analyseur de données (9) est configuré pour estimer un acide gras lié à une position sn-2 de glycérol sur la base d'une intensité de signal d'une crête d'ion produit provenant de la chaîne aliphatique.

9. Spectromètre de masse selon la revendication 1, le spectromètre de masse étant configuré pour effectuer une mesure d'un lipide en tant qu'échantillon cible, et le spectromètre de masse comprenant en outre :
un analyseur de données (9) configuré pour estimer un site de liaison saturée et un site de liaison insaturée sur la base d'intensités de signal de crêtes d'ions produits sur un spectre de masse créé sur la base de signaux de détection obtenus par la section de séparation-détection (3, 4, 107, 108) .

10. Spectromètre de masse selon la revendication 1, le spectromètre de masse étant configuré pour effectuer une mesure d'un lipide en tant qu'échantillon cible, et le spectromètre de masse comprenant en outre :
un dissociateur d'ions utilisant des électrons configuré pour favoriser une dissociation d'un ion précurseur en irradiant l'ion précurseur avec des électrons, l'ion précurseur étant isolé par l'isolateur d'ion précurseur (2, 104) sans irradier les ions avec les espèces radicalaires ; et
un analyseur de données (9) configuré pour estimer un site de liaison insaturée sur la base d'un décalage de valeur de masse d'une crête provenant du même ion produit sur un spectre de masse obtenu lorsque l'ion précurseur est dissocié par le dissociateur d'ions utilisant des électrons et un spectre de masse obtenu lorsque l'ion précurseur est isolé et dissocié après la réaction avec l'espèce radicalaire.

11. Procédé d'analyse de lipide utilisant la spectrométrie de masse, comprenant :
a) une étape de réaction d'ions dans laquelle des ions provenant d'un échantillon cible incluant un lipide sont irradiés avec des espèces radicalaires d'une molécule prédéterminée pour induire une réaction entre les ions et les espèces radicalaires, dans lequel les espèces radicalaires de la molécule prédéterminée incluent au moins un type d'espèces radicalaires choisies parmi un radical hydrogène, un radical hydroxyle et un radical oxygène, dans lequel une irradiation avec des radicaux hydrogène induit une réaction d'extraction d'hydrogène, et dans lequel une irradiation avec des radicaux hydroxyle ou des radicaux oxygène induit une réaction d'extraction d'hydrogène ou de fixation d'oxygène ;
b) une étape d'isolation d'ion précurseur dans laquelle un ion spécifique qui s'est transformé en un ion radicalaire par ladite réaction est sélectivement isolé en tant qu'ion précurseur, de sorte que les ions produits générés en raison de la dissociation par irradiation des ions avec les espèces radicalaires sont éliminés ;
c) une étape de dissociation d'ions dans laquelle l'ion précurseur isolé dans l'étape d'isolation d'ion précurseur est dissocié pour générer des ions produits ;
d) une étape de séparation-détection dans laquelle les ions produits générés dans l'étape de dissociation d'ions sont séparés les uns des autres selon leurs rapports masse/charge et détectés en tant que signaux de détection ; et
e) une étape d'analyse de données dans laquelle un spectre de masse créé sur la base des signaux de détection obtenus dans l'étape de séparation-détection est recherché pour une paire de crêtes présentant une différence de masse caractéristique d'un site de liaison insaturée d'une chaîne aliphatique, et une position du site de liaison insaturée dans le lipide est déterminée sur la base de la paire de crêtes localisée.

12. Procédé d'analyse de lipide selon la revendication 11, dans lequel :
l'étape d'analyse de données inclut la recherche d'une paire de crêtes présentant une différence de masse de 12 Da.
